# EUROPEAN PATENT SPECIFICATION

(11) **EP 2 587 331 B1**
(45) Date of publication and mention of the grant of the patent: **31.05.2017**
(21) Application number: 11186765.1
(22) Date of filing: 26.10.2011
(51) Int. Cl.: G05D 1/08, A61B 5/11, A61B 5/00

(54) **Method for direction changes identification and tracking**
Verfahren zur Identifikation und Verfolgung von Richtungsänderungen
Procédé d'identification et de suivi de changement de direction

(43) Date of publication of application: 01.05.2013
(73) Proprietor: Sony Mobile Communications Inc., Shinagawa-ku Tokyo 140-0002 (JP)
(72) Inventor: Persson, Magnus, 247 47 Flyinge (SE); Hansson, Anton, 452 38 Strömstad (SE); Tufvesson, Linus, 226 46 Lund (SE)
(74) Representative: Neij & Lindberg AB

(56) References cited:
- EP-A1- 0 109 784
- US-A- 5 050 087
- US-A1- 2007 032 748
- CHRISTIAN LUKIANTO ET AL: "Pedestrian smartphone-based indoor navigation using ultra portable sensory equipment", INDOOR POSITIONING AND INDOOR NAVIGATION (IPIN), 2010 INTERNATIONAL CONFERENCE ON, IEEE, PISCATAWAY, NJ, USA, 15 September 2010 (2010-09-15), pages 1-5, XP031809256, ISBN: 978-1-4244-5862-2
- NISARG KOTHARI ET AL: "Robust Indoor Localization on a Commercial Smart-Phone", TECH. REPORT CMU-RI-TR-11-27, CARNEGIE MELLON UNIVERSITY, 1 August 2011 (2011-08-01), page 11PP, XP009154616,

## Description

### TECHNICAL FIELD

The invention relates in general to the field of navigation, and more particularly, to the determination and tracking of the heading of a moving person using a mobile communication device.

### BACKGROUND

Using modern mobile communication devices, such as mobile phones, for position tracking of a moving object such as a person or a car has become very popular. Outdoors, the global positioning system (GPS) is often used as the primary way of determining parameters such as current position, speed and heading of an object. However, indoors the GPS signal is often very weak or none existing, making it impossible to determine the position, speed and heading of a person with a mobile communication device with any accuracy.

A major concern when constructing a navigation system for tracking movement of a person, without having access to any reference signal such as a GPS signal, is how to accurately determine the heading of the person. A common way of tracking the heading is to use a three-axis gyroscope, and assume that all angular velocity changes registered by the gyroscope correspond to heading changes of the moving object. However, the angular velocity changes measured by the gyroscope do not necessarily correspond to heading changes of the moving object. If for instance a moving object, such as a person walking, is holding a mobile phone in his hand it could be hard to distinguish angular velocity due to heading changes of the person from angular velocity due to movement of the hand holding the mobile
communication device. Thus, finding a way to improve the determination and tracking of the heading of a moving object is therefore highly sought after.

### SUMMARY OF THE INVENTION

With the above description in mind, then, an aspect of the present invention is to provide a way to improve the determination and tracking of the heading of a moving object which seeks to mitigate, alleviate, or eliminate one or more of the above-identified deficiencies in the art and disadvantages singly or in any combination.
As will be described in more detail by the aspects of the present invention below, one way to improve the tracking and determination of the heading of a moving object is to use multiple (at least two) spatial separated sensors at the moving object to measure its angular velocity, and then to use the measurements to calculate and determine an accurate heading of the moving object, according to the aspects of the present invention below.

A first aspect of the present invention relates to a system for determining the heading of a moving object, wherein the system comprising, a first sensor adapted to register angular velocity and to generate a first signal in response to said registered angular velocity, at least a second sensor adapted to register at least a second angular velocity and to generate at least a second signal in response to said registered at least second angular velocity, a processor adapted to receive said first signal and said at least a second signal, and wherein said first sensor and said processor is housed in a mobile communication device, and wherein said mobile communication device and said at least a second sensor are located at said moving object, and wherein said first sensor and said at least a second sensor are placed on different parts on said moving object such that they will generate different signal characteristics, and wherein said processor is further adapted to determine the heading of said moving object based on common features of said first signal and said at least second signal.

The system wherein said at least a second sensor may be housed in at least an accessory device, and wherein said at least an accessory device is located at said moving object and at a distance from said mobile communication device.

The system wherein said first sensor and said at least a second sensor may be gyroscope sensors.

The system wherein said accessory device may be any of: a second mobile communication device, a wired headset, a wireless headset, a wristwatch, a necklace, glasses, an ear piece, a shoe, a glove, or a piece of clothing.

The system wherein said mobile communication device may be a mobile phone.

The system wherein said processor may be further adapted to receive said at least a second signal either by wire or wirelessly.

A second aspect of the present invention relates to a mobile communication device comprising a display adapted to display information related to said determined heading of a said moving object.

A third aspect of the present invention relates to a method for determining the heading of a moving object, wherein the method comprising, determining, in a first sensor, a first angular velocity and generating a first signal based on said determined first angular velocity, transmitting said first signal to a processor, determining, in at least a second sensor, at least a second angular velocity and generating at least a second signal based on said determined at least a second angular velocity, transmitting said at least a second signal to said processor, receiving said first and said at least a second signal in said processor, wherein said processor and said first sensor are housed in a mobile communication device and wherein said mobile communication device and said at least a second sensors are located at said moving object, and wherein said first sensor and at least a second sensor are placed at different parts on said moving object such that they will generate different signal characteristics; and wherein said method further comprises determining the heading of said moving object based on common features of said received first signal and said at least a second signal.

The method wherein said transmission of said at least a second signal may be done either by wire or wirelessly.

The method wherein said first sensor and said at least a second sensor may be gyroscope sensors.

The variants presented in conjunction with the first, second and third aspect of the invention above may be combined in any way possible to form different variants of the embodiment of the present invention.

EP 0 109 784 A1 discloses a strapped-down inertial system for a vehicle including computational means and a sensor package. The sensor package is fixedly mounted on a turntable mounted on the frame of the vehicle.

US 5 050 087 A discloses a system and method for providing accurate attitude measurements at remote locations within an aircraft.

US 2007/032748 A1 a portable sensor system for detecting and analyzing body movement.

The article "Pedestrian smartphone-based indoor navigation using ultra-portable equipment" by Christian Lukianto et al discloses a system comprising as smartphone and portable INS device. The INS device consist of highly integrated MEMS sensors: Accelerometer and angular rate sensor which is supported by a barometric pressure sensor.

The article "Robust Indoor Localization on a Commercial Smart-Phone" by Nisage Kothari et al discloses a smart-phone based indoor localization system using dead-reckoning and wifi RSSI fingerprinting that is precise enough for giving way-finding directions and for use in context-aware applications.

### BRIEF DESCRIPTION OF THE DRAWINGS

Further objects, features, and advantages of the present invention will appear from the following detailed description of some embodiments of the invention, wherein some embodiments of the invention will be described in more detail with reference to the accompanying drawings, in which:
Fig. 1 shows a system for determining the heading of a moving object, according to an embodiment of the present invention; and
Fig. 2 shows a flowchart describing the steps in a method for determining the heading of a moving object, according to an embodiment of the present invention; and
Fig. 3 shows different places to place sensors for determining the heading of a moving object on a person, according to an embodiment of the present invention.

### DETAILED DESCRIPTION

Embodiments of the present invention will be described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art.

Embodiments of the present invention will be exemplified using a mobile phone and accessories thereof. However, it should be appreciated that the invention is as such equally applicable to any type of mobile communication device with heading tracking capabilities. Examples of such devices may for instance be any type off hand-held navigation devices, laptops (such as standard, ultra portables, netbooks, micro laptops, and pads), handheld computers, PDAs, tablets, pads, gaming devices, accessories to mobile phones, etc. However, for the sake of clarity and simplicity, the embodiments outlined in this specification are exemplified with and related to mobile phones and accessories thereof only.

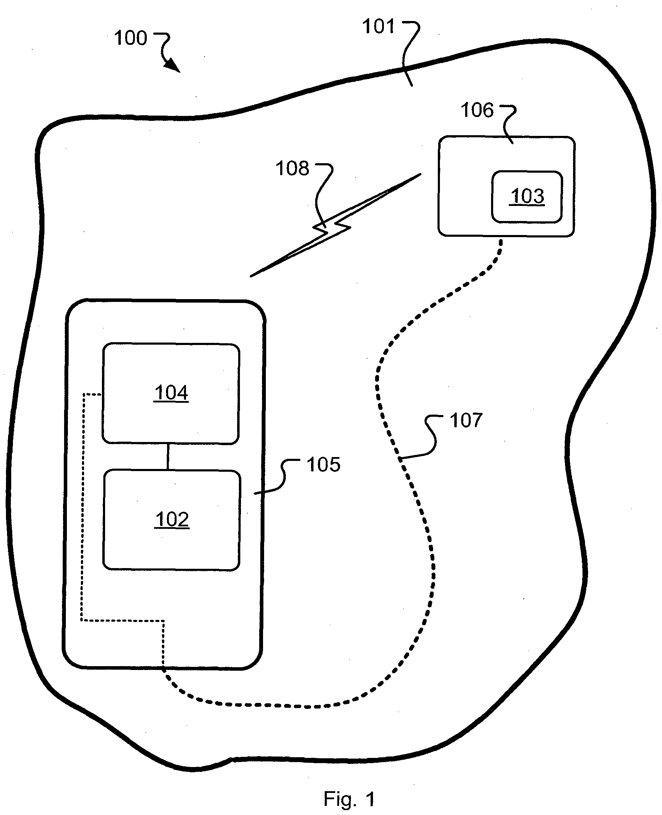

Figure 1 shows a block diagram of a system 100 which may be used to more accurately determine the heading of a moving object 101. A moving object 101 may for instance be a person, a vehicle, an airplane, a missile, a robot or any other type of object capable of moving in a direction. The system is comprised of a first sensor 102 capable of register angular velocity and a second sensor 103 also capable or registering angular velocity. The first sensor 102 generates a first signal in response to the registered angular velocity, and the second sensor 103 generates a second signal in response to said registered angular velocity. A typical sensor capable of registering an angular velocity and generating a signal in response thereof is a gyroscope.

A gyroscope is a device for measuring angular velocity (or orientation) based on the principles of conservation of angular momentum. Gyroscopes may be implemented in many different ways using different technologies. There are for instance mechanical gyroscope having a spinning wheel or disk whose axle is free to take any orientation, electronic MEMS gyroscope devices, solid-state ring laser gyroscopes, fiber optic gyroscopes, and the extremely sensitive quantum gyroscope. The discussed sensors 102 and 103 may be of any type of gyroscope mentioned above.

The two sensors 102,103 may preferably be placed at the moving object 101 (meaning either on, in or in the direct vicinity of the object 101) in question, and placed in such way that the sensors react (meaning registering angular velocity changes) to movement of the object 101.

The registered signals from the fist 102 and the second 103 sensor may be transmitted to a processor 104. The processor 104 may be adapted to determine the heading of the moving object 101 by doing calculations based on the received first 102 and second 103 sensor signals. Preferably the two sensors 102,103 are spaced apart from each other on the person's body (moving object 101), for example one sensor in the mobile phone 105 and one sensor at (attached or on) the person's head.

If the processor, in an variant of an embodiment of the present invention, is located away from the sensors 102,103 (or even away from the moving object 101), the sensors 102,103 then preferably transmits their signals to the processor by wireless communication 108. In another variant of an embodiment of the present invention the processor and the first and the second sensors may both, or at least one of them, be attached to the processor by wire 107.

In an embodiment of the present invention the processor 104 and the first sensor 102 is housed in a mobile communication device 105, in this case a mobile phone, and connected to each other by wire. The second sensor 103 is housed in an accessory device 106 and communicated with the processor 104 in the mobile phone 105 by wireless communication 108 or alternatively (hence the jagged line) by wire 107. Both the mobile phone 105 and the accessory device 106 must placed at the moving object 101 (i.e. be worn by the person) in order to generate an accurate determination of the moving object 101 (i.e. person's heading).

Preferably the mobile phone 105 and the accessory device 106 is spaced well apart on the persons body 101 (moving object). By spacing the two sensors 102,103 spatially apart from each other the processor can be programmed to analyze the two signals in relation to each other and thereby eliminate registered velocity changes which are not a result from an actual change in heading. Two sensors spaced well apart will generate signals that have common features, such as a true change in heading, and features which are not in common with each other, which in this case are referred to as heading noise. Some body parts are also more likely to move in the direction of the person's true heading, such as the torso, while other body parts such as the arms and hands may move in many different headings during a short period of time (for example a hand holding a mobile phone with a sensor which moves around in different directions when walking). Thus it may be preferable to position the mobile phone 105 and the accessory device 106 well apart and on body parts that are as stable (heading wise) as possible. However, since one of the sensors are located in an accessory device, the device may be restricted to be placed at certain positions since the accessory is mainly used in that or those position.

In a variant of the present invention the accessory device is a wireless headset 106 with an incorporated gyroscope (the gyroscope being the second sensor 103) worn in the person's ear. The mobile phone 105 is usually worn in one of the pockets of the person's trousers or in the person's hand. In this way the two sensors 102,103 are spaced well apart and the characteristic motions of the walking (registered by the first sensor 102 in the mobile phone 105) moving and the head bobbing (registered by the second sensor 103 in the accessory device 106) can easily be differentiated and recognized when determining the heading in the processor, and thus a more accurate heading may be determined in comparison to using only one sensor 102 in the mobile phone.

In yet another variant the accessory device 106 in the form of a headset may for instance be worn around the neck as a necklace, and the mobile phone may be worn in one of the pockets in the trousers or in the person's hand. Also in this case the motions of the body and the hand may easily be differentiated and recognized when determine the heading of the person.

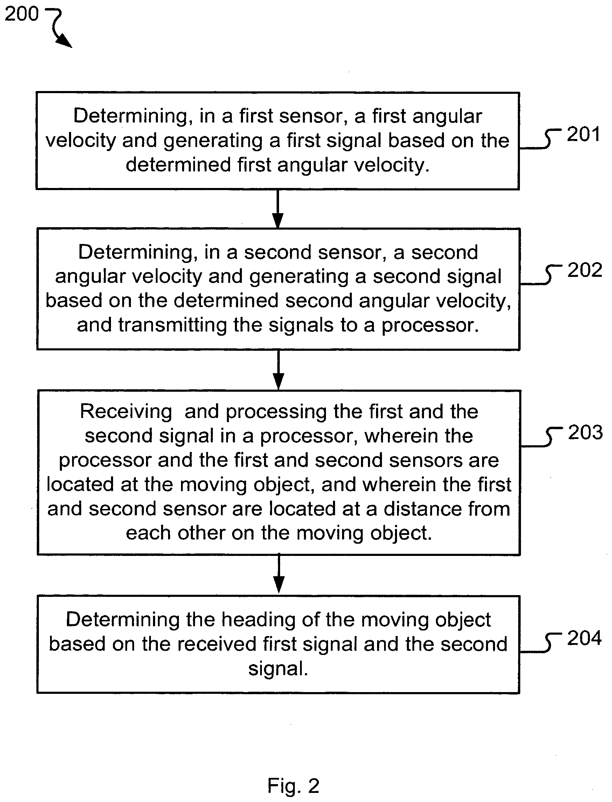

Figure 2 shows a flowchart describing the main steps in a method to more accurately determine the heading of a moving object 200 according to the system described above. The first step 201 is to determine in a first sensor, a first angular velocity and generate a first signal based on the determined first angular velocity. The second step 202 is to determine, in a second sensor, a second angular velocity and generate a second signal based on the determined second angular velocity. The generated first signal and the second signal are then transmitted to a processor. In the third step 203 the first and the second signal is received and processed in the processor, wherein the processor and the first and second sensors are located at the moving object, and wherein the first and second sensor are located at a distance from each other on the moving object. In the last step 204 of the method 200, the heading of the moving object is determined based on the received first signal and the second signal as discussed above. In this way it is possible to drastically improve the tracking of the heading of a moving object using a mobile phone 105 with a gyroscope and an accessory device 106 such as a headset with a second gyroscope. It should be noted that the method 200 described above and in the flowchart in figure 2 is not limited to only incorporating two sensors. It should be understood that the skilled person easily may extend the method to incorporate three or more sensors determining angular velocities of the moving object and communicate their signals to the processor to be incorporated into the determination of the heading of the moving object.

An accessory device 106 which incorporates a sensor, such as a gyroscope, capable of register angular velocity may be any type of a wired headset, a wireless headset, a wristwatch, a necklace, glasses, an ear piece, a shoe, a glove, and a piece of clothing. An accessory device 106 may also be a second mobile communication device. In all these devices a gyroscope may be integrated which registers angular velocity and transmit a signal, which is based on the registered angular velocity, to a reception device with a processor, such as a mobile phone.

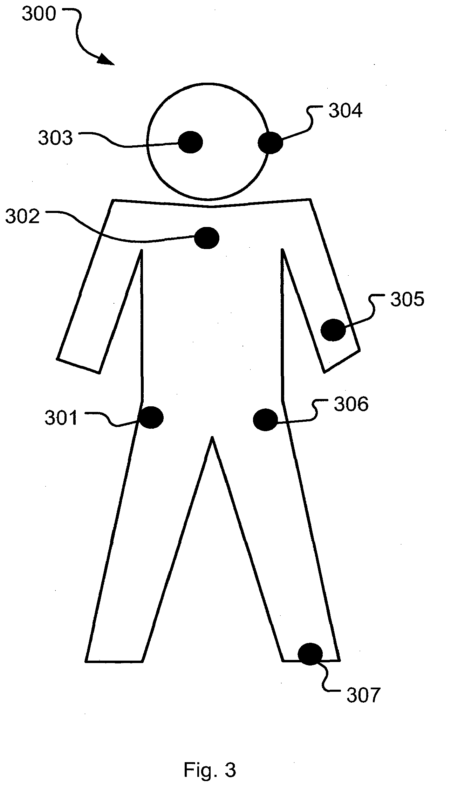

Figure 3 illustrates an outline of a person 300. The different dots 301-307 indicate different positions on the body where different mobile phone accessories such as wired headset, a wireless headset, a wristwatch, a necklace, glasses, an ear piece, shoes, gloves or even a second communication device usually may be located. It may be an advantage to locate one sensor at the waist 301,306 in a front or back pocket, on one of the wrists 305 or on one of the foot/legs 307, while placing the other sensor at the torso 302, by the eye 303 (in for example glasses) or on the head or in the ear 304. In this way we will get a good spatial separation of the sensors, and the sensors will be placed on locations on the body that will generate different signal characteristics. Sensors may also be incorporated into the fabric of the cloths worn by the person. In a variant, three or more sensors may be placed on different parts of the person's body to increase the accuracy of the determination of the heading of the person.

The present invention as described above may be used as a stand-alone system for determining the heading of a moving object, or it may be a part of a much larger navigation system. Especially an inertial navigation system may benefit from using the present invention described above.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" "comprising," "includes" and/or "including" when used herein, specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this invention belongs. It will be further understood that terms used herein should be interpreted as having a meaning that is consistent with their meaning in the context of this specification and the relevant art and will not be interpreted in an idealized or overly formal sense unless expressly so defined herein.

The foregoing has described the principles, preferred embodiments and modes of operation of the present invention. However, the invention should be regarded as illustrative rather than restrictive, and not as being limited to the particular embodiments discussed above. The different features of the various embodiments of the invention can be combined in other combinations than those explicitly described. It should therefore be appreciated that variations may be made in those embodiments by those skilled in the art without departing from the scope of the present invention as defined by the following claims.

## Claims

1. A system (100) for determining the heading of a moving object (101), wherein the system comprising;
- a first sensor (102) adapted to register angular velocity and to generate a first signal in response to said registered angular velocity;
- at least a second sensor (103) adapted to register at least a second angular velocity and to generate at least a second signal in response to said registered at least second angular velocity;
- a processor (104) adapted to receive said first signal and said at least a second signal; and
**characterized in that** said first sensor (102) and said processor (104) is housed in a mobile communication device (105), and wherein said mobile communication device (105) and said at least a second sensor (103) are located at said moving object (101), and wherein said first sensor (102) and said at least a second sensor (103) are placed on different parts on said moving object (101) such that they will generate different signal characteristics, and wherein said processor (104) is further adapted to determine the heading of said moving object (101) based on common features of said first signal and said at least second signal.

2. The system (100) according to claim 1, wherein said at least a second sensor (103) is housed in at least an accessory device (106), and wherein said at least an accessory device (106) is located at said moving object (101) and at a distance from said mobile communication device (105).

3. The system (100) according to any of the previous claims, wherein said first sensor (102) and said at least a second sensor (103) are gyroscope sensors.

4. The system (100) according to claim 3, wherein said accessory device (106) is any of: a second mobile communication device, a wired headset, a wireless headset, a wristwatch, a necklace, glasses, an ear piece, a shoe, a glove, or a piece of clothing.

5. The system (100) according to claims 1 - 4, wherein said mobile communication device (105) is a mobile phone.

6. The system (100) according to any of the previous claims, wherein said processor (104) is further adapted to receive said at least a second signal either by wire (107) or wirelessly (108).

7. The system (100) according to any of the previous claims, wherein the mobile communication device (105) comprising;
- a display adapted to display information related to said determined heading of a said moving object (101).

8. A method for determining the heading of a moving object (200), wherein the method comprising;
- determining (201), in a first sensor (102), a first angular velocity and generating a first signal based on said determined first angular velocity;
- transmitting (204) said first signal to a processor (104);
- determining (202), in at least a second sensor (103), at least a second angular velocity and generating at least a second signal based on said determined at least a second angular velocity;
- transmitting (204) said at least a second signal to said processor (104);
- receiving (203) said first and said at least a second signal in said processor (104),
**characterized in that** said processor and said first sensor are housed in a mobile communication device (105) and wherein said mobile communication device (105) and said at least a second sensors are located at said moving object (200), and wherein said first sensor (102) and at least a second sensor (103) are placed at different parts on said moving object such that they will generate different signal characteristics; and wherein said method further comprises:
- determining (204) the heading of said moving object based on common features of said received first signal and said at least a second signal.

9. The method according to claim 8, wherein said transmission of said at least a second signal is done either by wire (107) or wirelessly (108).

10. The method according to any of claims 8 - 9, wherein said first sensor (102) and said at least a second sensor (103) are gyroscope sensors.

## Patentansprüche

1. System (100) zum Bestimmen des Kurses eines sich bewegenden Objekts (101), wobei das System Folgendes umfasst:
- einen ersten Sensor (102), der dafür ausgelegt ist, eine Winkelgeschwindigkeit aufzuzeichnen und ein erstes Signal in Reaktion auf die aufgezeichnete Winkelgeschwindigkeit zu erzeugen;
- wenigstens einen zweiten Sensor (103), der dafür ausgelegt ist, wenigstens eine zweite Winkelgeschwindigkeit aufzuzeichnen und wenigstens ein zweites Signal in Reaktion auf die aufgezeichnete wenigstens eine zweite Winkelgeschwindigkeit zu erzeugen; und
- einen Prozessor (104), der dafür ausgelegt ist, das erste Signal und das wenigstens eine zweite Signal zu empfangen; und
**dadurch gekennzeichnet, dass** der erste Sensor (102) und der Prozessor (104) in einer Mobilkommunikationsvorrichtung (105) untergebracht sind und wobei die Mobilkommunikationsvorrichtung (105) und wenigstens ein zweiter Sensor (103) sich bei dem sich bewegenden Objekt (101) befinden und wobei der erste Sensor und der wenigstens eine zweite Sensor (103) in unterschiedlichen Teilen an dem sich bewegenden Objekt (101) angeordnet sind, so dass sie unterschiedliche Signaleigenschaften erzeugen, und wobei der Prozessor (104) ferner dafür ausgelegt ist, den Kurs des sich bewegenden Objekts (101) anhand gemeinsamer Merkmale des ersten Signals und des wenigstens einen zweiten Signals zu bestimmen.

2. System (100) nach Anspruch 1, wobei der wenigstens eine zweite Sensor (103) in wenigstens einer Zusatzvorrichtung (106) untergebracht ist und wobei sich die wenigstens eine Zusatzvorrichtung (106) bei dem sich bewegenden Objekt (101) und in einem Abstand von der Mobilkommunikationsvorrichtung (105) befindet.

3. System (100) nach einem der vorhergehenden Ansprüche, wobei der erste Sensor (102) und der wenigstens eine zweite Sensor (103) Gyroskopsensoren sind.

4. System (100) nach Anspruch 3, wobei die Zusatzvorrichtung (106) eine der Folgenden ist: eine zweite Mobilkommunikationsvorrichtung, ein drahtgebundener Kopfhörer, ein drahtloser Kopfhörer, eine Handgelenkuhr, ein Halsband, eine Brille, eine Hörmuschel, ein Schuh, ein Handschuh oder ein Kleidungsstück.

5. System (100) nach den Ansprüchen 1-4, wobei die Mobilkommunikationsvorrichtung (105) ein Mobiltelephon ist.

6. System (100) nach einem der vorhergehenden Ansprüche, wobei der Prozessor (104) ferner dafür ausgelegt ist, das wenigstens eine zweite Signal entweder drahtgebunden (107) oder drahtlos (108) zu empfangen.

7. System (100) nach einem der vorhergehenden Ansprüche, wobei die Mobilkommunikationsvorrichtung (105) Folgendes umfasst:
- eine Anzeige, die dafür ausgelegt ist, Informationen, die mit dem bestimmten Kurs des sich bewegenden Objekts (101) in Beziehung stehen, anzuzeigen.

8. Verfahren zum Bestimmen des Kurses eines sich bewegenden Objekts (200), wobei das Verfahren Folgendes umfasst:
- Bestimmen (201) in einem ersten Sensor (102) einer ersten Winkelgeschwindigkeit und Erzeugen eines ersten Signals anhand der bestimmten ersten Winkelgeschwindigkeit;
- Senden (204) des ersten Signals zu einem Prozessor (104);
- Bestimmen (202) in wenigstens einem zweiten Sensor (103) wenigstens einer zweiten Winkelgeschwindigkeit und Erzeugen wenigstens eines zweiten Signals anhand der bestimmten wenigstens einen zweiten Winkelgeschwindigkeit;
- Senden (204) des wenigstens einen zweiten Signals zu dem Prozessor (104); und
- Empfangen (203) des ersten und des wenigstens einen zweiten Signals in dem Prozessor (104),
**dadurch gekennzeichnet, dass** der Prozessor und der erste Sensor in einer Mobilkommunikationsvorrichtung (105) untergebracht sind, wobei sich die Mobilkommunikationsvorrichtung (105) und der wenigstens eine zweite Sensor bei dem sich bewegenden Objekt (200) befinden und wobei der erste Sensor (102) und wenigstens ein zweiter Sensor (103) an unterschiedlichen Teilen an dem sich bewegenden Objekt angeordnet sind, so dass sie unterschiedliche Signaleigenschaften erzeugen; und wobei das Verfahren ferner Folgendes umfasst:
- Bestimmen (204) des Kurses des sich bewegenden Objekts anhand gemeinsamer Merkmale des empfangenen ersten Signals und des empfangenen wenigstens einen zweiten Signals.

9. Verfahren nach Anspruch 8, wobei das Senden des wenigstens einen zweiten Signals entweder drahtgebunden (107) oder drahtlos (108) erfolgt.

10. Verfahren nach einem der Ansprüche 8-9, wobei der erste Sensor (102) und der wenigstens eine zweite Sensor (103) Gyroskopsensoren sind.

## Revendications

1. Système (100) de détermination du cap d'un objet (101) en mouvement, le système comportant :
- un premier capteur (102) prévu pour enregistrer une vitesse angulaire et pour générer un premier signal en réponse à ladite vitesse angulaire enregistrée ;
- au moins un deuxième capteur (103) prévu pour enregistrer au moins une deuxième vitesse angulaire et pour générer au moins un deuxième signal en réponse à ladite ou auxdites deuxièmes vitesses angulaires enregistrées ;
- un processeur (104) prévu pour recevoir ledit premier signal et ledit ou lesdits deuxièmes signaux ; et **caractérisé en ce que** ledit premier capteur (102) et ledit processeur (104) sont logés dans un dispositif mobile (105) de communications, et ledit dispositif mobile (105) de communications et ledit ou lesdits deuxièmes capteurs (103) sont situés au niveau dudit objet (101) en mouvement, et ledit premier capteur (102) et ledit ou lesdits deuxièmes capteurs (103) sont placés sur des parties différentes sur ledit objet (101) en mouvement de telle façon qu'ils génèrent des caractéristiques de signal différentes, et ledit processeur (104) étant en outre prévu pour déterminer le cap dudit objet (101) en mouvement d'après des traits communs dudit premier signal et dudit ou desdits deuxièmes signaux.

2. Système (100) selon la revendication 1, ledit ou lesdits deuxièmes capteurs (103) étant logés dans au moins un dispositif accessoire (106), et ledit ou lesdits dispositifs (106) d'accessoires étant situés au niveau dudit objet (101) en mouvement et à une certaine distance dudit dispositif mobile (105) de communications.

3. Système (100) selon l'une quelconque des revendications précédentes, ledit premier capteur (102) et ledit ou lesdits deuxièmes capteurs (103) étant des capteurs à gyroscope.

4. Système (100) selon la revendication 3, ledit dispositif (106) d'accessoire étant un dispositif quelconque parmi : un deuxième dispositif mobile de communications, un casque filaire, un casque sans fil, une montre-bracelet, un collier, des lunettes, une oreillette, une chaussure, un gant ou un article d'habillement.

5. Système (100) selon les revendications 1 à 4, ledit dispositif mobile (105) de communications étant un téléphone mobile.

6. Système (100) selon l'une quelconque des revendications précédentes, ledit processeur (104) étant en outre prévu pour recevoir ledit ou lesdits deuxièmes signaux soit par fil (107), soit sans fil (108).

7. Système (100) selon l'une quelconque des revendications précédentes, le dispositif mobile (105) de communications comportant :
- un affichage prévu pour afficher des informations liées audit cap déterminé dudit objet (101) en mouvement.

8. Procédé de détermination du cap d'un objet (200) en mouvement, le procédé comportant les étapes consistant à :
- déterminer (201), dans un premier capteur (102), une première vitesse angulaire et générer un premier signal d'après ladite première vitesse angulaire déterminée ;
- envoyer (204) ledit premier signal à un processeur (104) ;
- déterminer (202), dans au moins un deuxième capteur (103), au moins une deuxième vitesse angulaire et générer au moins un deuxième signal d'après ladite ou lesdites deuxièmes vitesses angulaires déterminées ;
- envoyer (204) ledit ou lesdits deuxièmes signaux audit processeur (104) ;
- recevoir (203) ledit premier et ledit ou lesdits deuxièmes signaux dans ledit processeur (104),
**caractérisé en ce que** ledit processeur et ledit premier capteur sont logés dans un dispositif mobile (105) de communications et ledit dispositif mobile (105) de communications et ledit ou lesdits deuxièmes capteurs étant situés au niveau dudit objet (200) en mouvement, et ledit premier capteur (102) et ledit ou lesdits deuxièmes capteurs (103) étant placés sur des parties différentes sur ledit objet en mouvement de telle façon qu'ils génèrent des caractéristiques de signal différentes ; et ledit procédé comportant en outre l'étape consistant à :
- déterminer (204) le cap dudit objet en mouvement d'après des traits communs dudit premier signal reçu et dudit ou desdits deuxièmes signaux.

9. Procédé selon la revendication 8, ledit envoi dudit ou desdits deuxièmes signaux étant effectué soit par fil (107), soit sans fil (108).

10. Procédé selon l'une quelconque des revendications 8 à 9, ledit premier capteur (102) et ledit ou lesdits deuxièmes capteurs (103) étant des capteurs à gyroscope.
